# EUROPÄISCHE PATENTSCHRIFT

(11) **EP 3 886 744 B1**
(45) Veröffentlichungstag und Bekanntmachung des Hinweises auf die Patenterteilung: **28.05.2025**
(21) Anmeldenummer: 19813758.0
(22) Anmeldetag: 28.11.2019
(51) Int. Cl.: A61B 17/80

(54) **MEDIZINISCHES KETTENNETZ**
MEDICAL CHAIN NET
FILET À CHAÎNES MÉDICAL

(30) Priorität: 28.11.2018 DE 102018130205
(43) Veröffentlichungstag der Anmeldung: 06.10.2021
(73) Patentinhaber: Aesculap AG, 78532 Tuttlingen (DE)
(72) Erfinder: HETTICH, Georg, 78532 Tuttlingen (DE); SCHWANZ, Lena, 78333 Stockach (DE)
(74) Vertreter: Winter, Brandl - Partnerschaft mbB
(86) Internationale Anmeldenummer: PCT/EP2019/082963
(87) Internationale Veröffentlichungsnummer: WO 2020/109501

(56) Entgegenhaltungen:
- WO-A1-2008/061759
- US-A1- 2018 271 572
- US-B2- 10 039 651

## Beschreibung

### Technisches Gebiet

Die vorliegende Offenbarung betrifft ein medizinisches Produkt, vorzugsweise in Form eines Kettennetzes zur Anwendung bei der Behandlung, insbesondere beim Auffüllen und/oder Verschluss, eines Knochendefekts, mit einer Vielzahl von Einzelgliedern, welche derart miteinander verbunden sind, dass zueinander benachbarte Einzelglieder ineinandergreifen/ineinander verkettet sind.

Ab einer gewissen Größe verheilen knöcherne Defekte am menschlichen Skelett nicht intrinsisch. Das heißt, die Defekte können nicht von innen heraus mit eigenem Antrieb geheilt werden, sondern benötigen eine medizinische Versorgung. In den letzten Jahren haben sich unterschiedliche Lösungen zur Versorgung von knöchernen Defekten etabliert. Vor allem bei Revisionen nach einer totalen Hüft- oder Kniearthroplastik (Hüft-oder Kniegelenkersatz) besteht häufig der Bedarf, kavitäre Knochendefekte aufzufüllen. Unter einer Revision versteht man im medizinischen Bereich die erneute, in der Regel chirurgische, Behandlung nach einer bereits erfolgten Therapie. Die Auffüllung von Knochendefekten ist teilweise auch im Bereich der Wirbelsäulen- und Traumachirurgie erforderlich. Insbesondere bei osteoporotischen und tumorbefallenen Knochen gestaltet sich die Auffüllung von kavitären Knochendefekten jedoch häufig schwierig.

Unter dem Ausdruck kavitärer Knochendefekt und/oder Knochenkavität ist ein Hohlraum oder eine Aushöhlung in einem menschlichen oder tierischen Knochen, insbesondere in einem menschlichen oder tierischen Gelenksknochen zu verstehen. Hierbei kann der Hohlraum die Folge eines Knochentraumas, einer Knochenerkrankung oder einer chirurgischen Intervention/Reintervention, insbesondere einer Revision nach einer totalen Hüft- oder Kniearthroplastik, sein.

Aus dem Stand der Technik sind verschiedene Behandlungsoptionen bekannt. Beispielsweise offenbart die DE 9 2013 226 063 A1 ein medizinisches Produkt zur Anwendung bei der Behandlung einer Knochenkavität, wobei das Produkt eine Mehrzahl von miteinander verbundenen Gliedern aufweist, wobei jedes Glied eine umlaufende Berandung aufweist und die Berandungen benachbarter Glieder ineinandergreifen.

Um einen knöchernen Defekt aufzufüllen werden oft formbare und an den Knochen anpassbare Füllmaterialien wie zum Beispiel Calziumphosphatzemente verwendet. Um eine Gitterstruktur für das Knochenwachstum bereitzustellen werden oftmals sogenannte Scaffolds verwendet. Diese Scaffolds sind vorgeformt und starr und passen sich nicht an den Knochen an.

Im Allgemeinen wird als Scaffold (im Deutschen "Gerüst") ein bioresorbierbarer Stent bezeichnet, der sich in einem Zeitraum von 24 Monaten stufenweise abbaut.

Für die Abdeckung von Knochendefekten am Acetabulum werden Metallnetze verwendet, welche eine geringe Flexibilität aufweisen. Acetabulum ist eine Hüftgelenk- oder Beckenpfanne und bildet in der Anatomie den vom Becken gebildeten knöchernen Anteil des Hüftgelenks. Vorgeformte, metallische Implantate zur Überbrückung eines acetabulären Knochendefekts, insbesondere Revisionsnetze, sind beispielsweise "Noviomagus Revision Meshes" von Spierings, "X-Change Revision Mesh" von Stryker. Flexible Gitterstrukturen sind bekannt als 3D -gedruckte Textilgewebe (Cellular Textiles) und Mesostructured Cellular Materials.

Die Noviomagus Revisionsnetze wurden für die Eindämmung von Knochentransplantaten bei der Durchführung von Impaktionsknochentransplantationen entwickelt, um die anatomische Form und die Abmessungen während der Hüftrevisionschirurgie wiederherzustellen. Diese sterilen Implantate aus Edelstahl haben eine anatomische Form, um eine korrekte Anpassung an die Hüftpfanne oder das proximale Femur zu gewährleisten. Obwohl diese Implantate so konzipiert sind, dass sie der allgemeinen menschlichen Anatomie entsprechen, lassen sie sich leicht an den individuellen Patienten anpassen. Eine derartige Anpassung ist lediglich durch Zuschneiden von Schlitzen an strategischen Positionen vorgesehen.

Des Weiteren ist in der Anmeldung WO 2015 91 518 A1 ein Titan-Netz zur Revision offenbart, das zur Behebung eines Knochendefekts bei einer Kniegelenksersatzoperation verwendet wird. Das Titan-Netz zur Revision hat Querrippenriemen zum Implantieren des Titan-Netzes zur Revision in einem menschlichen Körper und Längsrippenriemen zum Tragen der Querrippenriemen. Die Querrippenriemen und die Längsrippenriemen werden gekreuzt und zu einem maschenförmigen Titan-Netz zur Revision kombiniert.

Des Weiteren ist in der Anmeldung EP 0 89 883 A2 ein Netz zum Überbrücken einer Lücke in einem Knochen offenbart, das aus einem biokompatiblen Material besteht und eine Struktur hat, die aus mehreren Maschenpunkten am Ende von bogenförmigen Schnüren besteht. Jede ihrer Öffnungen, die einen Durchmesser unter 1 mm hat, kann eine Schiene aufnehmen und die Öffnungen ermöglichen das Entfernen von Knochenschrauben.

Des Weiteren ist in der Anmeldung US 2018/271 572 A1 eine miniatur- und mikroskalige konforme Kettenpostvorrichtung für die Skelettfixierung, Stabilisierung und Reparatur sowie Verfahren zu deren Herstellung und Verwendung beschrieben. Die strukturellen Vorrichtungen umfassen ein anpassungsfähiges Blatt aus miteinander verbundenen polygonalen Gliedern, die ein Kettengeflecht mit einer ersten und einer zweiten Außenfläche bilden. Dabei umfassen die miteinander verbundenen Glieder planare Oberflächen, die sich unter Bildung der ersten bzw. zweiten Außenfläche des anpassungsfähigen Blattes kombinieren. Ebenfalls vorgesehen sind Methoden zu Verwendung der Strukturvorrichtung zur Stabilisierung von Knochengewebe, zur Fixierung von Knochengewebe, als Knochentransplantatpflaster oder als dünner Knochengewebeersatz.

Der Nachteil der vorstehend beschriebenen Anmeldung ist, dass keine mechanische Stabilität ab einer definierten Krümmung gegeben ist und alle Glieder gleich ausgebildet sind.

Somit bieten die bisherigen Lösungen zu Behandlung von Knochendefekten entweder eine formbare Masse, ein komplett starres Scaffold/Revisionsnetz oder eine flexible Gitterstruktur ohne mechanische Stabilität für die Überbrückung eines Knochendefektes.

### Kurzbeschreibung der Erfindung

Der vorliegenden Erfindung liegt daher die Aufgabe zugrunde, ein medizinisches Produkt zur Behandlung von Knochendefekten bereitzustellen, welches anpassbare, flexible und doch in sich starre Scaffolds/Revisionsnetze sind, insbesondere um die Nachteile aus dem Stand der Technik zu verbessern.

Die Aufgabe der Erfindung wird dadurch gelöst, dass Einzelglieder des Kettennetzes in Verbindungsglieder und Hauptglieder unterteilt sind. Die Verbindungsglieder sind zu den Hauptgliedern unterschiedlich ausgebildet, wobei die Verbindungsglieder und die Hauptglieder im miteinander verketteten Zustand eine in x-y-Ebene flächige Gitterstruktur bilden.

Die Verkettung der Einzelglieder bietet die Möglichkeit ein flächiges Gitter/Netz auszubilden, welches zur flächigen Überbrückung eines Knochendefekts verwendet werden kann. Die Hauptglieder und die dazu unterschiedlich ausgebildeten Verbindungsglieder sind an sich durch eine lose Verbindung miteinander, insbesondere ineinander, verkettet, um so die flächige Gitterstruktur auszubilden und sind relativ zueinander, vorzugsweise begrenzt, beweglich.

Der Vorteil der verketteten Einzelglieder, die ein flächiges Implantat in der Anwendung Knochendefekte zu überbrücken bilden, ist, dass diese Überbrückung isoliert als Scaffold für Knochenwachstum oder als Abgrenzung für Knochendefektauffüllungen genutzt werden kann.

Ein vorteilhafter Aspekt der vorliegenden Erfindung ist, dass die Verbindungsglieder und die Hauptglieder dreidimensional ausgebildet sind, oder nur die Hauptglieder dreidimensional und die Verbindungsglieder zweidimensional ausgebildet sind.

Durch die Verwendung von zumindest zweidimensionalen Einzelgliedern, sind verschieden Kombinationsmöglichkeiten zwischen Hauptgliedern und Verbindungsgliedern gegeben, welche eine unterschiedliche Flexibilität beziehungsweise Anpassungsfähigkeit aufweisen können.

Des Weiteren ist es bevorzugt, wenn die flächige Gitterstruktur in z-Richtung krümmbar ist und die Hauptglieder in z-Richtung asymmetrisch ausgestaltet sind. Insbesondere durch die an sich lose Verbindung der Einzelglieder kann die flächige Gitterstruktur in z-Richtung gekrümmt werden.

Es ist von Vorteil, wenn die Hauptglieder eine maximale Krümmung der flächigen Gitterstruktur durch deren gegenseitige Berührung vordefinieren. Zum Anpassen des Scaffolds kann die gemäß dem ersten Aspekt in der x-y-Ebene ausgebildete Fläche sowohl in die positive als auch in die negative z-Richtung gekrümmt werden. Durch die asymmetrische Ausgestaltung der Hauptglieder in z-Richtung ergeben sich weitere Möglichkeiten, wie nachstehend beschrieben.

Ein vorteilhafter Aspekt der vorliegenden Erfindung ist, dass die Verbindungsglieder ein weiteres Bewegen der Hauptglieder, insbesondere ein Entfernen voneinander, beim Erreichen der vordefinierten maximalen Krümmung verhindern. Somit ist das medizinische Produkt, insbesondere aufgrund der aus Einzelgliedern aufgebauten Struktur, begrenzt deformierbar.

In anderen Worten, wird das in der x-y-Ebene liegende flächige Gitter/Netz in z-Richtung gekrümmt. Sobald eine bestimmte/vordefinierte Krümmung erreicht ist, berühren sich die Hauptglieder und verhindern eine weitere Krümmung der Gitterstruktur. Ist dieser Fall eingetreten, können sich die Hauptglieder aufgrund der Verbindungsglieder auch nicht wieder voneinander entfernen. Somit sorgt ein Zusammenspiel der Hauptglieder und der Verbindungsglieder für ein starres Gitterkonstrukt, sobald eine maximale Krümmung, welche der vordefinierten Krümmung entspricht, erreicht ist. Durch diese Vorteile kann das Implantat sich an den Knochen anpassen und trotzdem einen Knochendefekt mechanisch stabil überbrücken. Vor Erreichen der vordefinierten und gewünschten Krümmung bietet das Scaffold eine flexible Gitterstruktur, die für eine Anpassung ideal ausgebildet ist, und dann versteift.

In anderen Worten ist der Vorteil hierbei, dass eine Anpassung an den Knochen derart ermöglicht wird, dass das Implantat bis zu einer gewissen Krümmung flexibel ist und ab dem Überschreiten einer vordefinierten, insbesondere einstellbaren, Krümmung versteift und mechanisch belastbar ist.

Des Weiteren bietet die vorliegende Lösung mit Hilfe von verschieden geformten Hauptgliedern in Kombination mit verschieden geformten Verbindungsglieder, die Möglichkeit, unterschiedliche maximale Krümmungen zu definieren beziehungsweise einzustellen.

Weiter ist bevorzugt, wenn die vordefinierte maximale Krümmung in positive z-Richtung unterschiedlich zu der vordefinierten maximalen Krümmung in negative z-Richtung ist.

Dies wird durch die asymmetrische Ausgestaltung der Hauptglieder in z-Richtung ermöglicht. Somit kann das flächige Gitterkonstrukt in zwei unterschiedliche maximale Krümmungen gebracht werden und ist somit auf der einen Seite anderes definiert/eingestellt als auf der anderen Seite. Die Asymmetrie der Hauptglieder in z-Richtung bietet lediglich unterschiedlich viel Bewegungsfreiheit der gesamten Einzelgliederkombination, wodurch der Krümmungsradius festgelegt ist.

Beispielsweise wird bei der Versorgung eines Acetabulumdefekt ein maximaler Krümmungsradius in positive z-Richtung von 20 bis 30 mm verwendet und in negativer z-Richtung beträgt der maximale Krümmungsradius 30 bis 45 mm. Demnach beträgt ein relativer Unterschied im Krümmungsradius in positive z-Richtung 20% weniger als in negative z-Richtung.

Bei dem Anwendungsbeispiel des Scaffolds bei der Versorgung eines Schädelplattendefekts beträgt der maximale Krümmungsradius in positive z-Richtung 60 bis 60 mm und der maximale Krümmungsradius in negative z-Richtung 60 bis 90 mm. Demnach beträgt der relative Krümmungsradius in positive z-Richtung 9% weniger als in negative z-Richtung.

Vorteilhaft ist es, wenn die Hauptglieder und die Verbindungsglieder eine in sich geschlossene Form aufweisen. Des Weiteren weist jedes Hauptglied und jedes Verbindungsglied zumindest eine Öffnung, ein Loch/Aussparung auf, welches durch eine geschlossene Umrandung definiert ist. Zudem ist es vorteilhaft, wenn alle Hauptglieder und Verbindungsglieder abgerundete Kanten haben, und mögliche Spitzen abgeflacht sind, so dass auch der Rand der flächigen Gitterstruktur kein Verletzungspotential für umliegendes, insbesondere Gewebe hat. Des Weiteren erklärt sich von selbst, dass ein solches Implantat eine Befestigungsvorrichtung aufweisen kann, mit welcher das Scaffold an einem Knochen befestigt werden kann.

Gemäß einer ersten Ausführungsform ist es von Vorteil, wenn die Hauptglieder jeweils als ein fachwerkartiger, dreieckiger Pyramidenstumpf ausgebildet sind und die Verbindungsglieder jeweils als ein ösenförmiges Vieleck, insbesondere Sechseck, ausgebildet sind. Ein Hauptglied hat gemäß dieser Ausführungsform Kontakt mit drei Verbindungsgliedern, und ein Verbindungsglied hält sechs Hauptglieder zusammen, wobei eine Seitenkante eines jeden dreieckigen Pyramidenstumpfs in Richtung der Mitte des Verbindungsglieds zeigt.

Gemäß einer zweiten Ausführungsform ist bevorzugt, wenn die Hauptglieder als fachwerkartige, vorzugsweise verlängerte, viereckige Pyramiden ausgebildet sind und die Verbindungsglieder als eine kuppelförmige Gitterschale mit zumindest vier Bögen an der Basis und mit entsprechend zumindest vier (gesichtsförmigen) Aussparungen, welche jeweils mittig zwischen zwei benachbarten Bögen angeordnet sind und eine Unterkante der Aussparung sich auf der Höhe des Scheitelpunkts der Bögen befindet, ausgebildet sind.

Gemäß einer dritten Ausführungsform ist es vorteilhaft, wenn die Hauptglieder jeweils ein erstes als ösenförmiges Vieleck, insbesondere Sechseck ausgebildetes Grundelement, welches eine Ebene definiert, und ein zweites als ösenförmiges Vieleck, insbesondere als Sechseck ausgebildetes Grundelement haben, welches sich über zumindest eine Ebene erstreckt, die an den Ecken über orthogonale, insbesondere unterschiedlich lange Seitenkanten miteinander verbunden sind, und die Verbindungsglieder durch jeweils zumindest drei an der Basis miteinander verbundene Bögen ausgebildet sind.

In anderen Worten ausgedrückt besteht das Hauptglied in diesem bevorzugten Fall aus einem fachwerkartigen Vieleck-/Sechseckrohr, dessen End-/Stirnflächen miteinander abgekippt sind.

Gemäß einer vierten Ausführungsform ist bevorzugt, wenn die Hauptglieder jeweils durch eine Verbindung eines ersten und eines zweiten zentralen Punkts ausgebildet sind, die einander gegenüberliegen und mit zumindest vier nach außen gebogenen/geschwungenen Seitenkanten, insbesondere mit zumindest zwei benachbarten Seitenkanten, die eine größere Ausbuchtung näher an dem ersten zentralen Punkt und mit zumindest zwei weiteren benachbarten Seitenkanten, die eine größere Ausbuchtung näher an dem zweiten zentralen Punkt aufweisen, (kronenartig) verbunden sind (und somit die Grundform eines US-Footballs annehmen) und die Verbindungsglieder jeweils als zwei fachwerkartige, geschlossene, viereckige Pyramiden ausgebildet sind, welche an deren Unter-/Bodenseite zu einem geschlossen Körper miteinander verbunden sind.

Bei einem Krümmen des medizinischen Produkts gemäß einer der vorstehend beschriebenen Ausführungsformen in die positive z-Richtung, das heißt in dem Fall in Richtung der Oberseite des medizinischen Produkts, wird der Abstand zwischen den Kanten/Rändern der benachbarten Hauptglieder, die die Oberseite der Hauptglieder definieren größer und der Abstand zwischen den Kanten/Ränder der benachbarten Hauptglieder, die die Unterseite der Hauptglieder definieren kleiner. Sobald sich die Kanten der Hauptglieder, die die Unterseite der Hauptglieder definieren, berühren, ist die maximale, vordefinierte Krümmung erreicht und das medizinische Produkt versteift, beziehungsweise die Hauptglieder und Verbindungsglieder verhaken so miteinander, dass keine weitere Bewegung mehr möglich ist.

Bei einem Krümmen des medizinischen Produkts gemäß einer der vorstehend beschriebenen Ausführungsformen in die negative z-Richtung, das heißt in dem Fall in Richtung der Unterseite des medizinischen Produkts, wird der Abstand zwischen Kanten/Rändern der benachbarten Hauptglieder, die die Unterseite der Hauptglieder definieren größer und der Abstand zwischen den Kanten/Rändern der benachbarten Hauptglieder, die die Oberseite der Hauptglieder definieren kleiner. Sobald sich die Kanten der Hauptglieder, die die Oberseite der Hauptglieder definieren, berühren, ist die maximale, vordefinierte Krümmung erreicht und das medizinische Produkt versteift, beziehungsweise die Hauptglieder und Verbindungsglieder verhaken so miteinander, dass keine weitere Bewegung mehr möglich ist.

### Kurzbeschreibung der Figuren

Fig. 1 ist eine Darstellung des medizinischen Produkts gemäß einer ersten Ausführungsform der vorliegenden Offenbarung;
Fig. 2 ist eine Darstellung zur Veranschaulichung eines dreidimensionalen Hauptglieds gemäß der ersten Ausführungsform der vorliegenden Offenbarung;
Fig. 3 ist eine Darstellung zur Veranschaulichung eines zweidimensionalen Verbindungsglieds gemäß der ersten Ausführungsform der vorliegenden Offenbarung;
Fig. 4 ist eine ausschnittsweise Darstellung des medizinischen Produkts gemäß der ersten Ausführungsform der vorliegenden Offenbarung;
Fig. 5 ist eine Darstellung des medizinischen Produkts gemäß einer zweiten Ausführungsform der vorliegenden Offenbarung;
Fig. 6 ist eine Darstellung zur Veranschaulichung eines dreidimensionalen Hauptglieds gemäß der zweiten Ausführungsform der vorliegenden Offenbarung;
Fig. 7 ist eine Darstellung zur Veranschaulichung eines dreidimensionalen Verbindungsglieds gemäß der zweiten Ausführungsform der vorliegenden Offenbarung;
Fig. 8 ist eine ausschnittsweise Darstellung des medizinischen Produkts gemäß der zweiten Ausführungsform der vorliegenden Offenbarung;
Fig. 9 ist eine Darstellung des medizinischen Produkts gemäß einer dritten Ausführungsform der vorliegenden Offenbarung;
Fig. 10 ist eine Darstellung zur Veranschaulichung eines dreidimensionalen Hauptglieds gemäß der dritten Ausführungsform der vorliegenden Offenbarung;
Fig. 11 ist eine Darstellung zur Veranschaulichung eines dreidimensionalen Verbindungsglieds gemäß der dritten Ausführungsform der vorliegenden Offenbarung;
Fig. 12 ist eine ausschnittsweise Darstellung des medizinischen Produkts gemäß der dritten Ausführungsform der vorliegenden Offenbarung;
Fig. 13 ist eine Darstellung des medizinischen Produkts gemäß einer vierten Ausführungsform der vorliegenden Offenbarung;
Fig. 14 ist eine Darstellung zur Veranschaulichung eines dreidimensionalen Hauptglieds gemäß der vierten Ausführungsform der vorliegenden Offenbarung;
Fig. 15 ist eine Darstellung zur Veranschaulichung eines dreidimensionalen Verbindungsglieds gemäß der vierten Ausführungsform der vorliegenden Offenbarung;
Fig. 16 ist eine ausschnittsweise Darstellung des medizinischen Produkts gemäß der vierten Ausführungsform der vorliegenden Offenbarung;

### Beschreibung der Ausführungsbeispiele

Nachstehend werden Ausführungsbeispiele der vorliegenden Offenbarung auf der Basis der zugehörigen Figuren beschrieben. Die Figuren sind lediglich schematischer Natur und dienen dem Verständnis der Erfindung. Die gleichen Elemente sind mit denselben Bezugszeichen bezeichnet.

### Erste Ausführungsform

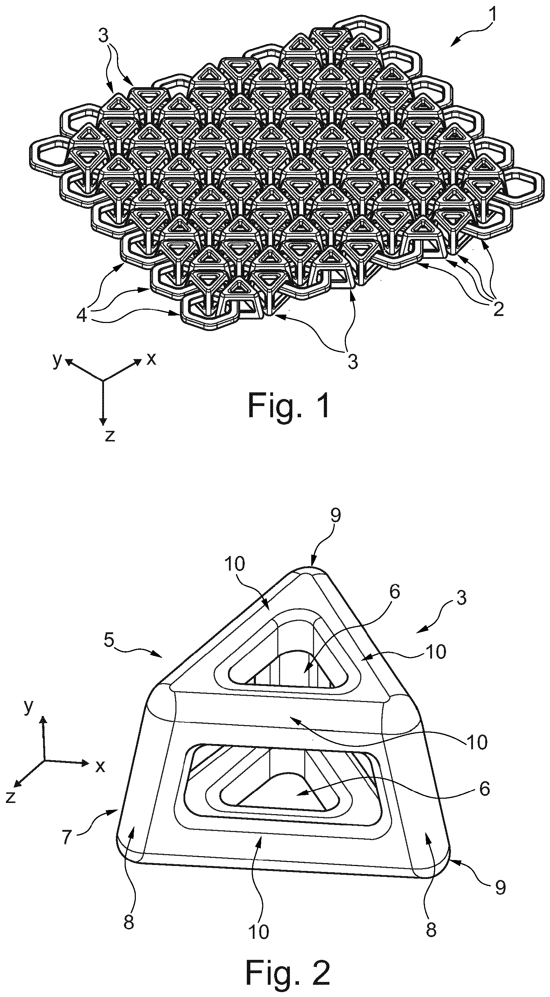

Fig. 1 ist eine Darstellung des medizinischen Produkts 1 gemäß einer ersten Ausführungsform. Das gezeigte medizinische Produkt 1 zeigt eine Vielzahl von Einzelgliedern 2, welche derart miteinander verbunden sind, dass zueinander benachbarte Einzelglieder 2 ineinander verkettet sind. Die Einzelglieder 2 können in Hauptglieder 3 und Verbindungsglieder 4 unterteilt/aufgeteilt werden, welche eine voneinander unterschiedliche Geometrie/Form aufweisen.

Durch die in Fig. 1 dargestellte Verkettung der Einzelglieder 2 beziehungsweise der Hauptglieder 3 und der Verbindungsglieder 4 entsteht ein flächiges Gitterkonstrukt, welches in der x-y-Ebene liegt und in die z-Richtung gekrümmt/gebogen werden kann.

In Fig. 2 ist zu erkennen, dass ein Hauptglied 3 in der Draufsicht eine dreieckige Form, insbesondere die Form eines gleichseitigen Dreiecks, hat. Eine Grundkante 10 eines ersten Hauptglieds 3 ist benachbart zu einer Grundkante 10 eines zweiten Hauptglieds 3. Bei einem derartigen Zusammenfügen der Hauptglieder 3, sind sechs Hauptglieder 3 jeweils durch ein Verbindungsglied 4 miteinander verbunden, sofern das Verbindungsglied 4 sich in der Mitte des medizinischen Produkts 1, insbesondere des Implantats, befindet. Ein Hauptglied 3, welches sich in der Mitte des medizinischen Produkts 1 befindet, hat Kontakt mit drei Verbindungsgliedern 4.

Fig. 2 ist eine Darstellung zur Veranschaulichung eines dreidimensionalen Hauptglieds 3 gemäß der ersten Ausführungsform der vorliegenden Offenbarung. Das Hauptglied 3 der ersten Ausführungsform ist ein fachwerkartiger, dreieckiger Pyramidenstumpf. In anderen Worten, hat das Hauptglied 3 ein erstes dreidimensionales Dreieck 5 mit einer ersten dreieckigen Aussparung 6 in dessen Mitte und ein zweites dreidimensionales Dreieck 7 mit einer zweiten dreieckigen Aussparung 6 in dessen Mitte. Das erste Dreieck 5 ist oberhalb von dem zweiten Dreieck 7 angeordnet und die Grundfläche des ersten Dreiecks 5 ist kleiner als die Grundfläche des zweiten Dreiecks 7. Die Spitzen 9 der beiden Dreiecke 5 und 7 sind über die drei Seitenkannten 8 des Pyramidenstumpfs mit einem an die verschiedenen Grundflächen angepassten Neigungswinkel miteinander verbunden.

Die Kanten und Ecken von dem in Fig. 2 als fachwerkartigen, dreieckigen Pyramidenstumpf dargestellten Hauptglied 3 sind abgerundet. Das Hauptglied 3 gemäß der ersten Ausführungsform ist in z-Richtung asymmetrisch, da das untere Dreieck 7 größer ist als das obere Dreieck 5, und ist lediglich y-achsensymmetrisch. Insbesondere können die Neigungswinkel der drei Seitenkanten 8 einander entsprechend gleich oder unterschiedlich sein, wodurch auch die Symmetrieverhältnisse beeinflusst werden.

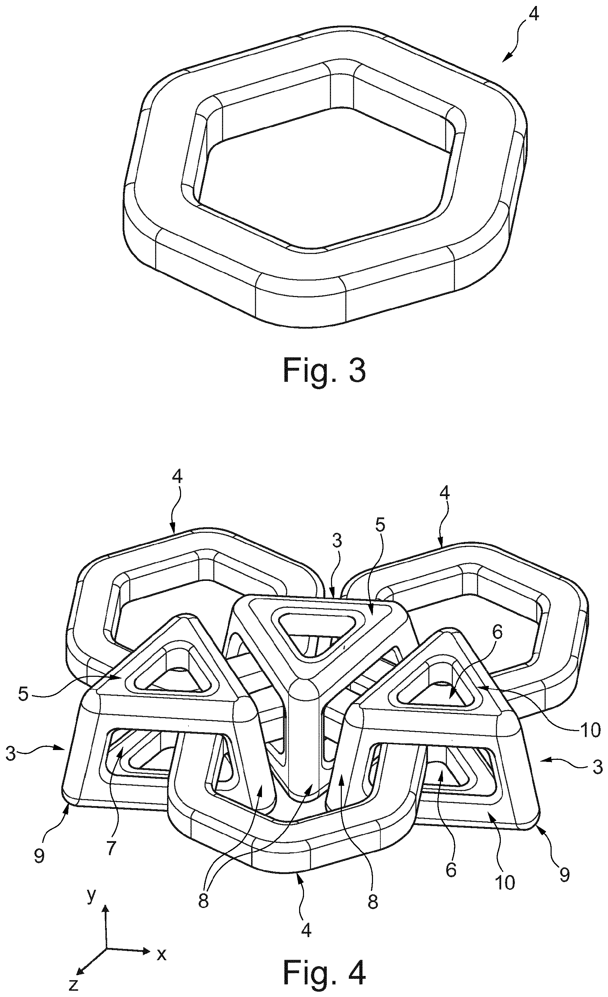

Fig. 3 ist eine Darstellung zur Veranschaulichung eines zweidimensionalen Verbindungsglieds 4 gemäß der ersten Ausführungsform der vorliegenden Offenbarung. Das Verbindungsglied 4 der ersten Ausführungsform ist beispielhaft als ein ösenförmiges Sechseck ausgebildet.

Auch das Verbindungsglied 4 hat abgerundete Kanten und Ecken. Das ösenförmige Sechseck weist über den gesamten Umfang eine gleichmäßige Dicke auf. Das Verbindungsglied 4 gemäß der ersten Ausführungsform ist sowohl x-, y- und z-achsensymmetrisch als auch punktsymmetrisch.

Fig. 4 ist eine ausschnittsweise Darstellung des medizinischen Produkts 1 gemäß der ersten Ausführungsform. Es ist zu erkennen, dass ein Hauptglied 3 mit drei Verbindungsgliedern 4 in Kontakt steht. Eine Ecke des dargestellten ösenförmigen Sechsecks dient zum Halten einer Seitenkante 8 des Pyramidenstrumpfs. In der ausschnittsweisen Darstellung ist gezeigt, dass aufgrund der Pyramidenstumpfform der Abstand zwischen den Grundseiten zweier benachbarter zweiter Dreiecke 7 geringer ist als der Abstand zwischen den Grundseiten zweier benachbarter erster Dreiecke 5.

Bei einem Krümmen des medizinischen Produkts 1 gemäß der vorstehend beschriebenen ersten Ausführungsformen in die positive z-Richtung, das heißt in dem Fall, die Krümmung des Implantats weist in Richtung der Oberseite des Pyramidenstumpfs, wird der Abstand zwischen den Grundkanten der benachbarten Dreiecke 5, die die jeweilige Oberseite der Pyramidenstümpfe definieren, größer und der Abstand zwischen den Grundkanten der benachbarten Dreiecke 7, die die jeweilige Unterseite der Pyramidenstümpfe definieren, kleiner. Sobald sich die Grundkanten der Dreiecke 5, die die jeweilige Unterseite der Pyramidenstümpfe definieren, berühren, ist die maximale, vordefinierte Krümmung erreicht und das medizinische Produkt 1 versteift, beziehungsweise die Hauptglieder 3 und Verbindungsglieder 4 verhaken so miteinander, dass keine weitere Bewegung mehr möglich ist.

Bei einem Krümmen des medizinischen Produkts 1 gemäß der vorstehend beschriebenen ersten Ausführungsformen in die negative z-Richtung, das heißt in dem Fall, die Krümmung des Implantats weist in Richtung der Unterseite des Pyramidenstumpfs, wird der Abstand zwischen den Grundkanten der benachbarten Dreiecke 5, die die jeweilige Oberseite der Pyramidenstümpfe definieren, kleiner und der Abstand zwischen den Grundkanten der benachbarten Dreiecke 7, die die jeweilige Unterseite der Pyramidenstümpfe definieren, größer. Sobald sich die Grundkanten der Dreiecke 7, die die jeweilige Oberseite der Pyramidenstümpfe definieren, berühren, ist die maximale, vordefinierte Krümmung erreicht und das medizinische Produkt 1 versteift, beziehungsweise die Hauptglieder 3 und Verbindungsglieder 4 verhaken so miteinander, dass keine weitere Bewegung mehr möglich ist.

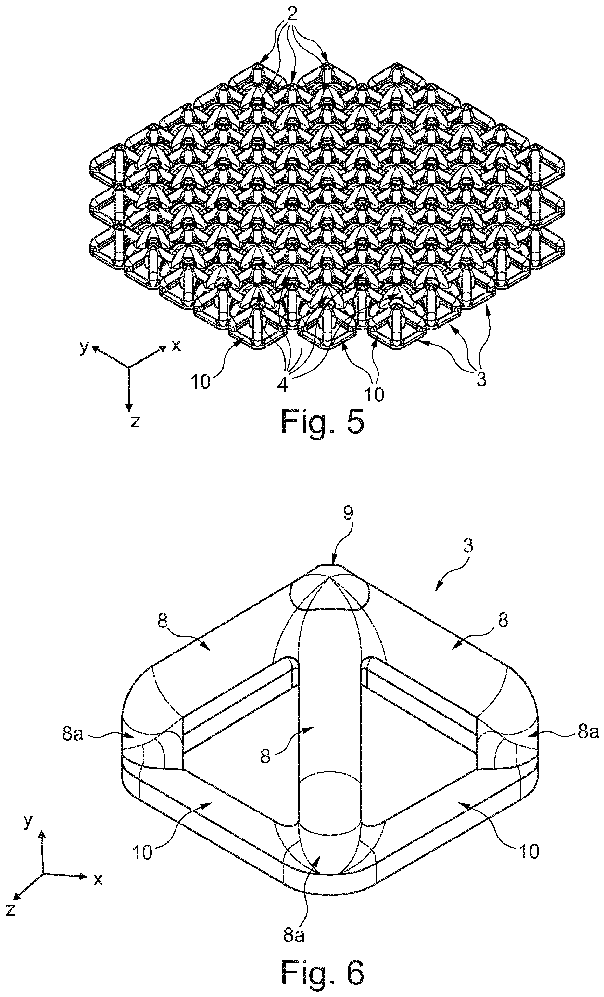

Fig. 5 ist eine Darstellung des medizinischen Produkts 1 gemäß einer zweiten Ausführungsform. Das gezeigte medizinische Produkt 1 zeigt eine Vielzahl von Einzelgliedern 2, welche derart miteinander verbunden sind, dass zueinander benachbarte Einzelglieder 2 ineinander verkettet sind. Auch diese Einzelglieder 2 sind wie in der ersten Ausführungsform in Hauptglieder 3 und Verbindungsglieder 4 unterteil/aufgeteilt, welche eine voneinander unterschiedliche Geometrie/Form aufweisen.

Durch die in Fig. 5 dargestellte Verkettung der Einzelglieder 2 beziehungsweise der Hauptglieder 3 und der Verbindungsglieder 4 entsteht ein flächiges Gitterkonstrukt, welches in der x-y-Ebene liegt und in die z-Richtung gekrümmt/gebogen werden kann.

In Fig. 5 ist zu erkennen, dass ein Hauptglied 3 in der Draufsicht eine vierseitige Pyramidenform hat. Des Weiteren ist die Pyramidenspitze 9 mit den Seitenkanten 8 zu sehen ist und die Seitenkanten 8 enden in den Ecken einer viereckigen Grundfläche. Eine Ansicht von unten zeigt aneinander gereihte Vierecke, die der Grundfläche des Hauptglieds 3 entsprechen. Eine Grundkante 10 eines ersten Hauptglieds 3 ist benachbart zu einer Grundkante 10 eines zweiten Hauptglieds 3. Bei einem derartigen Zusammenfügen der Hauptglieder 3, sind vier Hauptglieder 3 jeweils durch ein Verbindungsglied 4 miteinander verbunden, sofern das Verbindungsglied 4 sich in der Mitte des medizinischen Produkts 1, insbesondere des Implantats, befindet. Ein Hauptglied 3, welches sich in der Mitte des medizinischen Produkts 1 befindet, hat Kontakt mit vier Verbindungsgliedern 4. Die Ausbildung der Bögen 11 an den Verbindungsgliedern 4 dient zum Übergreifen über eine jeweilige Grundkante 10 des angrenzenden/benachbarten Hauptglieds.

Der Rand des medizinischen Produkts der zweiten Ausführungsform wird vorzugsweise durch Hauptglieder 3 ausgebildet.

Fig. 6 ist eine Darstellung zur Veranschaulichung eines dreidimensionalen Hauptglieds 3 gemäß der zweiten Ausführungsform der vorliegenden Offenbarung. Das Hauptglied 3 der zweiten Ausführungsform ist als eine fachwerkartige, verlängerte, viereckige Pyramide, nachfolgend als Enneaeder bezeichnet, ausgebildet. In anderen Worten, handelt es sich um eine fachwerkartige, viereckige Pyramide, wobei die vier Seitenkanten 8 der fachwerkartigen Pyramide über jeweils eine zur Boden-/Unterseite senkrecht stehende Seitenkante 8a in den Ecken der viereckigen Grundfläche enden. Die viereckige Grundfläche, ist ebenfalls fachwerkartig ausgebildet und bildet die vier Grundkanten 10.

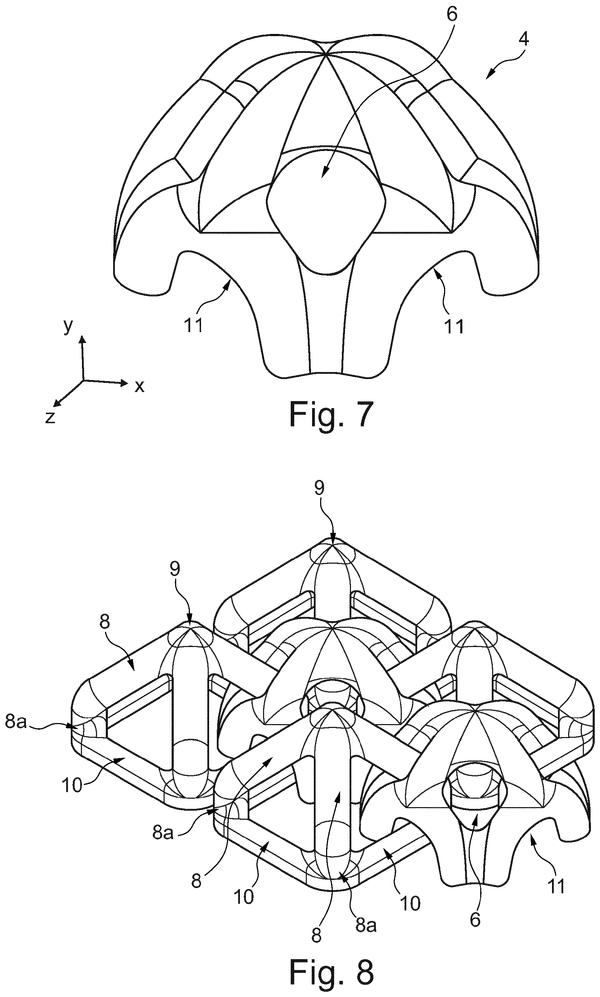

Fig. 7 ist eine Darstellung zur Veranschaulichung eines Verbindungsglieds 4 gemäß der zweiten Ausführungsform der vorliegenden Offenbarung. Das Verbindungsglied 4 ist als kuppelförmige Gitterschale mit zumindest vier Bögen 11 an der Basis und entsprechend zumindest vier ovale, insbesondere gesichtsförmige Aussparungen 6 jeweils mittig zwischen zwei benachbarten Bögen 11, wobei die Unterkante der Aussparung 6 auf Höhe des Scheitelpunkts der Bögen 11 liegt.

In anderen Worten, hat das Verbindungsglied 4 der zweiten Ausführungsform eine ähnliche Struktur eines Kreuzrippengewölbes mit vier Bögen 11 an der Basis mit jeweils einer ovalen, insbesondere gesichtsförmigen, Aussparung6 gemäß der vorstehenden Beschreibung. Dieser Struktur ähnelt in der Form und Struktur auch einem Pavillonzelt mit vier Standfüßen.

Die Kanten und Ecken, sowie die Spitze 9 des fachwerkartigen Enneaeders von dem in Fig. 6 dargestellten Hauptglied 3 und von dem in Fig. 7 dargestellten Verbindungsglied 4 sind abgerundet. Das Hauptglied 3 gemäß der zweiten Ausführungsform ist in z-Richtung asymmetrisch und ist lediglich y-achsensymmetrisch. Verbindungsglied 3 ist rotationssymmetrisch und weist eine Symmetrie ausgehend von x- und y-Achse auf.

Fig. 6 ist eine ausschnittsweise Darstellung des medizinischen Produkts 1 gemäß der zweiten Ausführungsform. Es ist zu erkennen, dass ein Hauptglied 3 mit vier Verbindungsgliedern 4 in Kontakt steht. Eine Seitenkante 8 des dargestellten Hauptglieds 3 führt durch eine Aussparung 6 des mit diesem in Kontakt stehenden Verbindungsglieds 4. Die Bögen 11 des Verbindungsglieds 4 dienen zum Übergreifen über eine Grundkante 10 des Enneaeders.

Bei einem Krümmen des medizinischen Produkts 1 gemäß der vorstehend beschriebenen ersten Ausführungsformen in die positive z-Richtung, das heißt in dem Fall, die Krümmung weist in Richtung der Oberseite des Enneaeders, wird der Abstand zwischen den Seitenkanten 8 der benachbarten Enneaeders, die die jeweilige Oberseite des Enneaeders definieren, größer und der Abstand zwischen den Grundkanten 10 der benachbarten Dreiecke 7, die die jeweilige Unterseite des Enneaeders definieren, kleiner. Sobald sich die Grundkanten 8, die die jeweilige Unterseite des Enneaeders definieren, berühren, ist die maximale, vordefinierte Krümmung erreicht und das medizinische Produkt 1 versteift, beziehungsweise die Hauptglieder 3 und Verbindungsglieder 4 verhaken so miteinander, dass keine weitere Bewegung mehr möglich ist.

Bei einem Krümmen des medizinischen Produkts 1 gemäß der vorstehend beschriebenen ersten Ausführungsformen in die negative z-Richtung, das heißt in dem Fall, die Krümmung weist in Richtung der Unterseite des Enneaeders, wird der Abstand zwischen den Seitenkanten 8 des Enneaeders, die die jeweilige Oberseite der Pyramidenstümpfe definieren, kleiner und der Abstand zwischen den Grundkanten 10 des Enneaeders, die die jeweilige Unterseite des Enneaeders definieren, größer. Sobald sich die Seitenkanten 8 des Enneaeders, die die jeweilige Oberseite des Enneaeders definieren, berühren, ist die maximale, vordefinierte Krümmung erreicht und das medizinische Produkt 1 versteift, beziehungsweise die Hauptglieder 3 und Verbindungsglieder 4 verhaken so miteinander, dass keine weitere Bewegung mehr möglich ist.

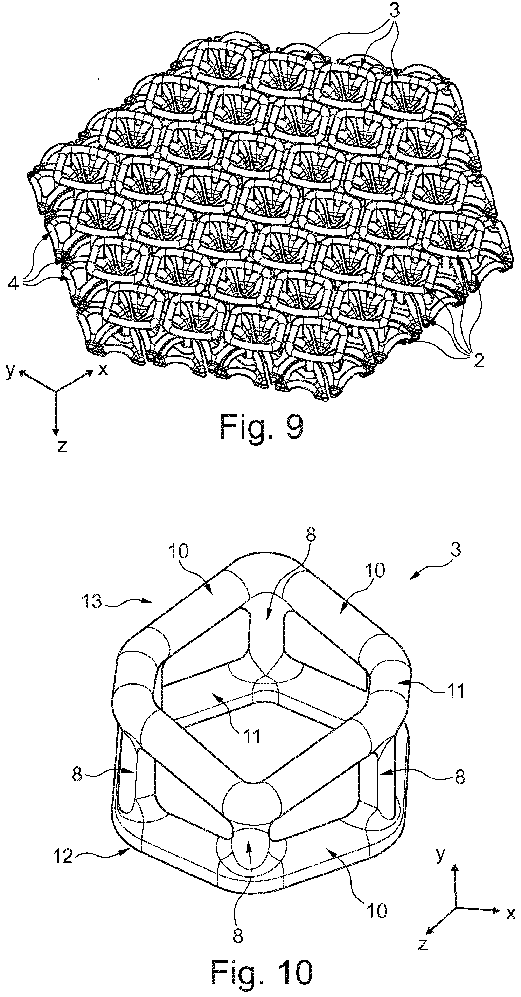

Fig. 9 ist eine Darstellung des medizinischen Produkts 1 gemäß einer dritten Ausführungsform. Fig. 9 ist eine Darstellung des medizinischen Produkts 1 gemäß einer dritten Ausführungsform. Das gezeigte medizinische Produkt 1 zeigt eine Vielzahl von Einzelgliedern 2, welche derart miteinander verbunden sind, dass zueinander benachbarte Einzelglieder 2 ineinander verkettet sind. Auch diese Einzelglieder 2 sind wie in der ersten und zweiten Ausführungsform in Hauptglieder 3 und Verbindungsglieder 4 unterteil/aufgeteilt, welche eine voneinander unterschiedliche Geometrie/Form aufweisen.

Durch die in Fig. 9 dargestellte Verkettung der Einzelelemente 2 beziehungsweise der Hauptglieder 3 und der Verbindungsglieder 4 entsteht ein flächiges Gitterkonstrukt, welches in der x-y-Ebene liegt und in die z-Richtung gekrümmt/gebogen werden kann.

In Fig. 9 ist zu erkennen, dass ein Hauptglied 3 in der Draufsicht ein unregelmäßiges Sechseck ist. Die Grundkanten 10 der sechseckigen Grundfläche eines Hauptglieds 3 liegen der Grundkanten 10 des benachbarten Hauptglieds 3 zugewandt. Bei einem derartigen Zusammenfügen der Hauptglieder 3, ist ein Hauptglied 3 jeweils durch sechs Verbindungsglied 4 miteinander verbunden, sofern das Verbindungsglied 4 sich in der Mitte des medizinischen Produkts 1, insbesondere des Implantats, befindet. Ein Verbindungsglied 4, welches sich in der Mitte des medizinischen Produkts 1 befindet, hat Kontakt mit drei Hauptgliedern 3.

Der Rand des medizinischen Produkts gemäß der zweiten Ausführungsform wird vorzugsweise durch Verbindungsglieder 4 ausgebildet.

Fig. 10 ist eine Darstellung zur Veranschaulichung eines dreidimensionalen Hauptglieds 3 gemäß der dritten Ausführungsform der vorliegenden Offenbarung. Das Hauptglied 3 hat jeweils ein erstes als ösenförmiges Vieleck, insbesondere Sechseck ausgebildetes Grundelement 12, welches in einer Ebene liegt, und ein zweites als ösenförmiges Vieleck, insbesondere als ein Sechseck, ausgebildetes Grundelement 13, welches sich über zumindest eine Ebene erstreckt. An den Ecken sind die beiden Grundelemente 12 und 13 über orthogonale, insbesondere unterschiedlich lange Seitenkanten 8 miteinander verbunden sind, wobei das Grundelement 13 das obere Grundelement ist.

In anderen Worten, handelt es sich bei der dritten Ausführungsform um zwei übereinander angeordnete Sechsecke, die mit unterschiedlich hohen/langen Seitenkanten 8 miteinander verbunden sind und die Seitenkanten 8 senkrecht zu dem unteren Grundelement 12 sind. Das obere Grundelement 13 ist an die unterschiedlich hohen Seitenkanten 8 angepasst und erstreckt sich daher über mehrere Ebenen, insbesondere drei Ebenen.

Gemäß der Darstellung in Fig. 10 ist es bevorzugt, wenn die kürzeste Seitenkante 8 der längsten Seitenkante 8 gegenüberliegend angeordnet ist und die vier verbleibenden Seitenkanten 8 gleich ausgebildet sind, jedoch kleiner als längste Seitenkante 8 und länger als die kürzeste Seitenkante 8.

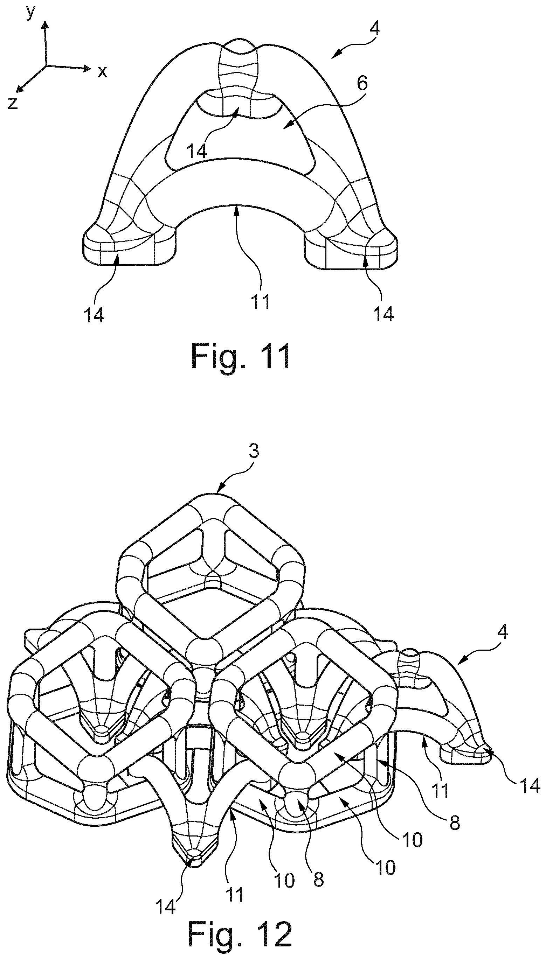

Fig. 11 ist eine Darstellung zur Veranschaulichung eines dreidimensionalen Verbindungsglieds 4 gemäß der dritten Ausführungsform der vorliegenden Offenbarung. Das Verbindungsglied 4 ist durch zumindest drei an der Basis miteinander verbundenen Bögen 11 ausgebildet. Die miteinander verbundenen Bögen 11 bilden in Richtung nach oben hin (positive z-Richtung) eine Öffnung, welche nachfolgend als Aussparung 6 bezeichnet ist. Die miteinander verbundenen Basen der drei Bögen 11 bilden jeweils ein Standbein 14 aus. Die zumindest drei Standbeine 14 spannen eine dreieckige Grundfläche auf und besitzen eine fußartige Ausbildung nach außen.

Die Kanten und Ecken sind, wie in den Figs. 10 und 11 gezeigt, abgerundet. Das Hauptglied 3 ist in z-Richtung asymmetrisch ausgebildet.

Fig. 12 ist eine ausschnittsweise Darstellung des medizinischen Produkts 1 gemäß der dritten Ausführungsform. Es ist zu erkennen, dass ein Hauptglied 3 mit sechs Verbindungsgliedern 4 in Kontakt steht. Jede Seitenkante 8 des dargestellten Hauptglieds 3 wird von einem mit diesem in Kontakt stehenden Verbindungsglied 4 umgriffen. Die Bögen 11 des Verbindungsglieds 4 dienen zum Übergreifen über eine Grundkante 10 des Hauptglieds 3. In anderen Worten, umgreift die Aussparung 6 eines Verbindungsglieds 4 jeweils eine Seitenkante 8 der drei benachbarten/angrenzenden Hauptglieder 3.

Bei einem Krümmen des medizinischen Produkts 1 gemäß der vorstehend beschriebenen dritten Ausführungsformen in die positive z-Richtung, das heißt in dem Fall, die Krümmung weist in Richtung des oberen Grundelements 13 des Hauptglieds 3, wird der Abstand zwischen den Grundkanten 10 der benachbarten Sechsecke, die das jeweilige obere Grundelement 13 des Hauptglieds 3 definieren, größer und der Abstand zwischen den Grundkanten 10 der benachbarten Sechsecke, die das jeweilige untere der Grundelement 12 definieren, kleiner. Sobald sich die Grundkanten 10 der Sechsecke, die das jeweilige untere Grundelement 12 definieren, berühren, ist die maximale, vordefinierte Krümmung erreicht und das medizinische Produkt 1 versteift, beziehungsweise die Hauptglieder 3 und Verbindungsglieder 4 verhaken so miteinander, dass keine weitere Bewegung mehr möglich ist.

Bei einem Krümmen des medizinischen Produkts 1 gemäß der vorstehend beschriebenen ersten Ausführungsformen in die negative z-Richtung, das heißt in dem Fall, die Krümmung weist in Richtung des unteren Grundelements 12 des Hauptglieds 3, wird der Abstand zwischen den Grundkanten 10 der benachbarten Sechsecke, die das jeweilige untere Grundelement 12 des Hauptglieds 3 definieren, kleiner und der Abstand zwischen den Grundkanten 10 der benachbarten Sechsecke, die das jeweilige untere Grundelement 12 des Hauptglieds 3 definieren, größer. Sobald sich die Grundkanten 10 der Sechsecke, die das jeweilige obere Grundelement 13 des Hauptglieds 3 definieren, berühren, ist die maximale, vordefinierte Krümmung erreicht und das medizinische Produkt 1 versteift, beziehungsweise die Hauptglieder 3 und Verbindungsglieder 4 verhaken so miteinander, dass keine weitere Bewegung mehr möglich ist.

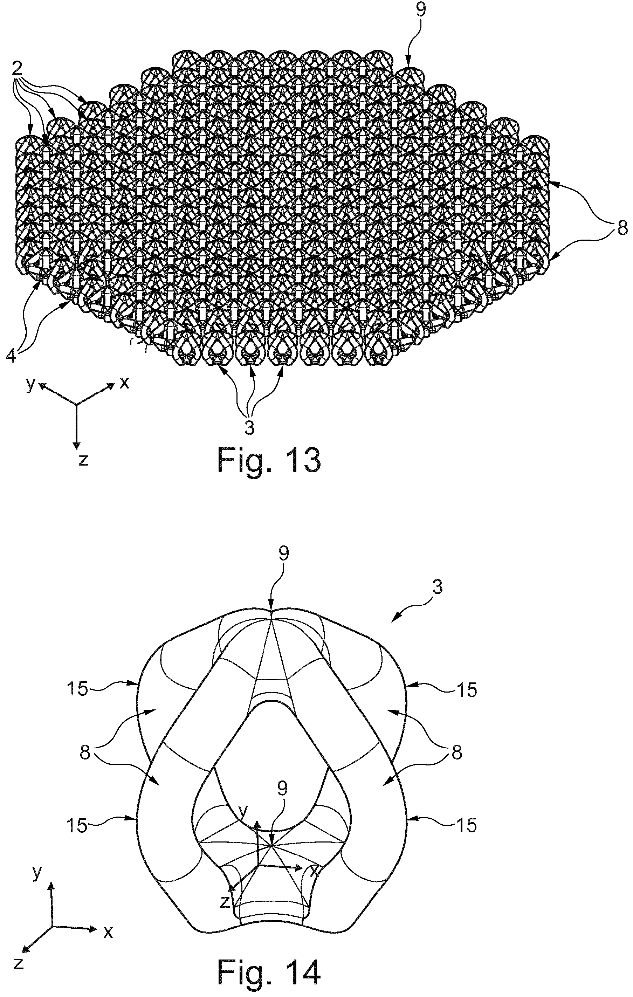

Fig. 13 ist eine Darstellung des medizinischen Produkts 1 gemäß einer vierten Ausführungsform. Fig. 13 ist eine ausschnittsweise Darstellung des medizinischen Produkts 1 gemäß der vierten Ausführungsform. Das gezeigte medizinische Produkt 1 zeigt eine Vielzahl von Einzelgliedern 2, welche derart miteinander verbunden sind, dass zueinander benachbarte Einzelglieder 2 ineinander verkettet sind. Die Einzelglieder 2 sind gemäß der ersten und zweiten Ausführungsform in Hauptglieder 3 und Verbindungsglieder 4 unterteilt/aufgeteilt, welche eine voneinander unterschiedliche Geometrie/Form aufweisen.

Durch die in Fig. 13 dargestellte Verkettung der Einzelglieder 2 beziehungsweise der Hauptglieder 3 und der Verbindungsglieder 4 entsteht ein flächiges Gitterkonstrukt, welches in der x-y-Ebene liegt und in die z-Richtung gekrümmt/gebogen werden kann.

In Fig. 13 sind in der Draufsicht lediglich pyramidenähnliche Spitzen 9 zu erkennen, von welchen Seitenkanten 8 in das flächige Gitterkonstrukt führen. Bei einer Betrachtung des medizinischen Produkts von der Unterseite, ergibt sich das gleiche Bild.

Fig. 14 ist eine Darstellung zur Veranschaulichung eines dreidimensionalen Hauptglieds 3 gemäß der vierten Ausführungsform der vorliegenden Offenbarung. Das Hauptglieder ist durch eine Verbindung eines ersten und eines zweiten zentralen Punkts 9 ausgebildet, die einander gegenüberliegen und mit zumindest vier nach außen gebogenen/geschwungenen Seitenkanten 8, insbesondere mit zumindest zwei benachbarten Seitenkanten 8, die eine größere Ausbuchtung 15 näher an dem ersten zentralen Punkt 9 und mit zumindest zwei weiteren benachbarten Seitenkanten 8, die eine größere Ausbuchtung 15 näher an dem zweiten zentralen Punkt 9 aufweisen, kronenartig verbunden sind. Somit befinden sich die Ausbuchtungen 15 vorzugsweise nicht in der Mitte der gebogenen/ geschwungenen Seitenkanten 8.

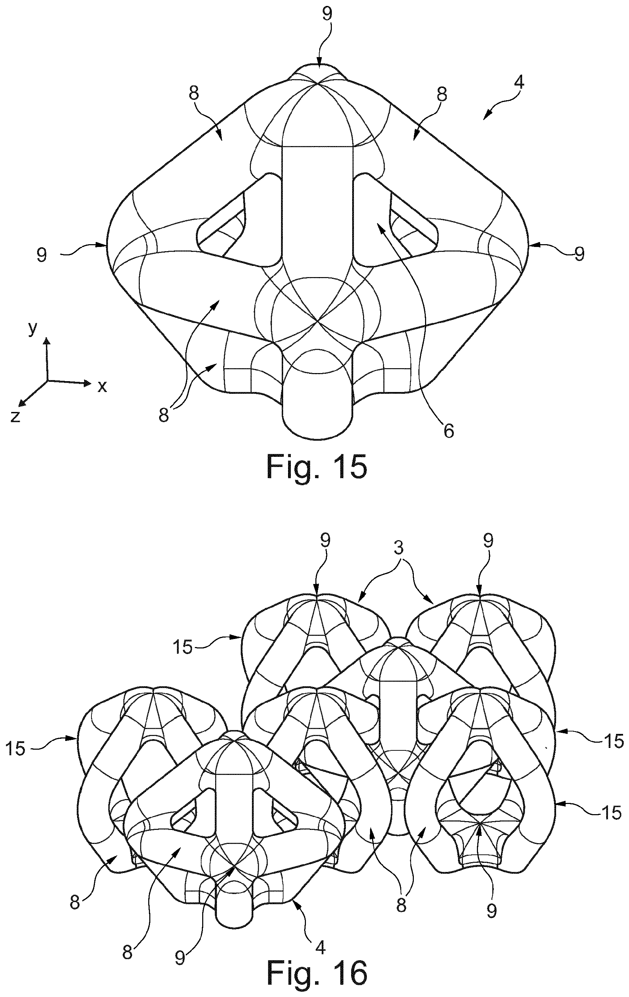

Fig. 15 ist eine Darstellung zur Veranschaulichung eines dreidimensionalen Verbindungsglieds 4 gemäß der vierten Ausführungsform der vorliegenden Offenbarung. Das Verbindungsglied 3 ist durch zwei fachwerkartige, geschlossene, viereckige Pyramiden ausgebildet sind, welche an der Unter-/Bodenseite zu einem geschlossen Körper miteinander verbunden sind.

In anderen Worten, ist das Verbindungsglied 4 der vierten Ausführungsform ein Polyeder, ausgebildet aus acht Dreiecksflächen, insbesondere gleichseitigen Dreiecksflächen, mit abgerundeten Ecken und Kanten, sowie mit einer dreieckigen Aussparungen 6 in jeder ausgebildeten Dreiecksfläche. Diese dreieckige Aussparung 6 ist vorzugsweise in ihrer Form an die Dreiecksfläche angepasst Die Umrandungen der dreieckigen Aussparungen 6 entsprechen den vorstehend beschrieben Seitenkanten 8. Vier zusammenlaufende Seitenkanten 8 bilden einen zentralen Punkt 9.

Die Ecken und Ränder des vorstehend beschriebenen Hauptglieds 3 und Verbindungsglied 4 haben abgerundete Ecken und Kanten. Das Hauptglied 3 gemäß Fig. 14 ist wiederum in z-Richtung asymmetrisch ausgebildet. Das Verbindungsglied 4 gemäß Fig. 15 ist x-, y- und z-Achsen symmetrisch, sowie punktsymmetrisch ausgerichtet.

Fig. 16 ist eine ausschnittsweise Darstellung des medizinischen Produkts 1 gemäß der vierten Ausführungsform der vorliegenden Offenbarung. Es ist zu erkennen, dass ein Hauptglied 3 mit vier Verbindungsgliedern 4 in Kontakt steht. Jede Seitenkante 8 des dargestellten Hauptglieds 3 ist durch zwei übereinander angrenzende Aussparungen 6 eines mit diesem in Kontakt stehenden Verbindungsglieds 4 geführt.

Bei einem Krümmen des medizinischen Produkts 1 gemäß der vorstehend beschriebenen dritten Ausführungsformen in die positive z-Richtung, das heißt in dem Fall, die Krümmung weist in Richtung des oberen zentralen Punkts 9 des Hauptglieds 3, wird der Abstand zwischen den geschwungenen Seitenkanten 8, die die jeweilige Oberseite des Hauptglieds 3 definieren, größer und der Abstand zwischen den geschwungenen Seitenkanten 8, die die jeweilige Unterseite des Hauptglieds 3 definieren, kleiner. Sobald sich die Seitenkanten 8 der Hauptglieder 3, die die jeweilige Unterseite des Hauptglieds 3 definieren, berühren, ist die maximale, vordefinierte Krümmung erreicht und das medizinische Produkt 1 versteift, beziehungsweise die Hauptglieder 3 und Verbindungsglieder 4 verhaken so miteinander, dass keine weitere Bewegung mehr möglich ist.

Bei einem Krümmen des medizinischen Produkts 1 gemäß der vorstehend beschriebenen dritten Ausführungsformen in die negative z-Richtung, das heißt in dem Fall, die Krümmung weist in Richtung des unteren zentralen Punkts 9 des Hauptglieds 3, wird der Abstand zwischen den geschwungenen Seitenkanten 8, die die jeweilige Oberseite des Hauptglieds 3 definieren, kleiner und der Abstand zwischen den geschwungenen Seitenkanten 8, die die jeweilige Unterseite des Hauptglieds 3 definieren, größer. Sobald sich die Seitenkanten 8 der Hauptglieder 3, die die jeweilige Unterseite des Hauptglieds 3 definieren, berühren, ist die maximale, vordefinierte Krümmung erreicht und das medizinische Produkt 1 versteift, beziehungsweise die Hauptglieder 3 und Verbindungsglieder 4 verhaken so miteinander, dass keine weitere Bewegung mehr möglich ist.

Insbesondere ist jede mögliche Kombination der vorstehend beschriebenen Hauptglieder und Verbindungsglieder denkbar.

### Bezugszeichenliste

- 1: Medizinisches Produkt
- 2: Einzelglied
- 3: Hauptglied
- 4: Verbindungsglied
- 5: erstes Dreieck
- 6: Aussparung
- 7: zweites Dreieck
- 8: Seitenkante
- 9: zentraler Punkt/Spitze
- 10: Grundkante
- 11: Bogen
- 12: erstes Grundelement
- 13: zweites Grundelement
- 14: Standbein
- 15: Ausbuchtung

## Patentansprüche

1. Medizinisches Produkt (1) insbesondere Implantat in Form eines Kettennetzes zur Anwendung bei der Behandlung, insbesondere beim Auffüllen und/oder Verschluss, eines Knochendefekts, mit einer Vielzahl von Einzelgliedern (2), welche derart miteinander verbunden sind, dass zueinander benachbarte Einzelglieder (2) ineinander verkettet sind, wobei
die Einzelglieder (2) des Kettennetzes in Hauptglieder (3) und Verbindungsglieder (4), unterteilt sind, wobei die Hauptglieder (3) und die Verbindungsglieder (4) im miteinander verketteten Zustand eine in x-y-Ebene flächige Gitterstruktur bilden, **dadurch gekennzeichnet, dass** die Verbindungsglieder (4) eine zu den Hauptgliedern (3) unterschiedliche geometrische Form aufweisen.

2. Medizinisches Produkt (1) gemäß Anspruch 1, **dadurch gekennzeichnet, dass** die Hauptglieder (3) und die Verbindungsglieder (4) dreidimensional ausgebildet sind, oder nur die Hauptglieder (3) dreidimensional und die Verbindungsglieder (4) zweidimensional ausgebildet sind.

3. Medizinisches Produkt (1) gemäß Anspruch 2, **dadurch gekennzeichnet, dass** die Hauptglieder (3) in z-Richtung asymmetrisch ausgestaltet sind.

4. Medizinisches Produkt (1) gemäß Anspruch 2 oder 3, **dadurch gekennzeichnet, dass** die flächige Gitterstruktur in z-Richtung krümmbar ist.

5. Medizinisches Produkt (1) gemäß Anspruch 4, **dadurch gekennzeichnet, dass** die Hauptglieder (3) eine maximale Krümmung der flächigen Gitterstruktur durch deren gegenseitige Berührung vordefinieren.

6. Medizinisches Produkt (1) gemäß Anspruch 5, **dadurch gekennzeichnet, dass** die Verbindungsglieder (4) ein weiteres Bewegen der Hauptglieder (3), insbesondere ein Entfernen voneinander, beim Erreichen der vordefinierten maximalen Krümmung verhindern.

7. Medizinisches Produkt (1) gemäß Anspruch 6, **dadurch gekennzeichnet, dass** die vordefinierte maximale Krümmung in positive z-Richtung unterschiedlich zu der vordefinierten maximalen Krümmung in negative z-Richtung ist.

8. Medizinisches Produkt (1) gemäß einem vorhergehenden Anspruch, **dadurch gekennzeichnet, dass** die Hauptglieder (3) und die Verbindungsglieder (4) eine in sich geschlossene Form aufweisen.

9. Medizinisches Produkt (1) gemäß Anspruch 6, **dadurch gekennzeichnet, dass** die Hauptglieder (3) jeweils als ein fachwerkartiger, dreieckiger Pyramidenstumpf ausgebildet sind und die Verbindungsglieder (4) jeweils als ösenförmiges Vieleck, insbesondere Sechseck, ausgebildet sind.

10. Medizinisches Produkt (1) gemäß Anspruch 6, **dadurch gekennzeichnet, dass** die Hauptglieder (3) als fachwerkartige, vorzugsweise verlängerte, viereckige Pyramiden ausgebildet sind und die Verbindungsglieder (4) als eine kuppelförmige Gitterschale mit zumindest vier Bögen (11) an der Basis und mit entsprechend zumindest vier Aussparungen (6), welche jeweils mittig zwischen zwei benachbarten Bögen (11) angeordnet sind und eine Unterkante der Aussparung (6) sich auf der Höhe des Scheitelpunkts der Bögen (11) befindet, ausgebildet sind.

11. Medizinisches Produkt (1) gemäß Anspruch 6, **dadurch gekennzeichnet, dass** die Hauptglieder (3) jeweils ein erstes als ösenförmiges Vieleck, insbesondere Sechseck ausgebildetes Grundelement (12), welches eine Ebene definiert, und ein zweites als ösenförmiges Vieleck, insbesondere als Sechseck ausgebildetes Grundelement (13) haben, welches sich über zumindest eine Ebene erstreckt, die an den Ecken über orthogonale, insbesondere unterschiedlich lange Seitenkanten (8) miteinander verbunden sind, und die Verbindungsglieder (4) durch jeweils zumindest drei an der Basis miteinander verbundene Bögen (11) ausgebildet sind.

12. Medizinisches Produkt (1) gemäß Anspruch 6, **dadurch gekennzeichnet, dass** die Hauptglieder (3) jeweils durch eine Verbindung eines ersten und eines zweiten zentralen Punkts (9) ausgebildet sind, die einander gegenüberliegen und mit zumindest vier nach außen gebogenen/geschwungenen Seitenkanten (8), insbesondere mit zumindest zwei benachbarten Seitenkanten (8), die eine größere Ausbuchtung (15) näher an dem ersten zentralen Punkt und mit zumindest zwei weiteren benachbarten Seitenkanten (8), die eine größere Ausbuchtung (15) näher an dem zweiten zentralen Punkt (9) aufweisen, verbunden sind und die Verbindungsglieder jeweils als zwei fachwerkartige, geschlossene, viereckige Pyramiden ausgebildet sind, welche an deren Unter-/Bodenseite zu einem geschlossen Körper miteinander verbunden sind.

## Claims

1. Medical product (1), in particular implant, preferably in the form of a chain net for use in the treatment, in particular in the filling and/or closure, of a bone defect, having a plurality of individual link elements (2) which are connected to each other in such a way that adjacent individual link elements (2) are linked to each other, wherein the individual link elements (2) of the chain net are subdivided into main link elements (3) and connection link elements (4), wherein the main link elements (3) and the connection link elements (4) form a grid structure which is planar in the x-y plane in the interlinked state, **characterized in that** the connection link elements (4) have a different geometric form from the main link elements (3).

2. Medical product (1) according to claim 1, **characterized in that** the main link elements (3) and the connection link elements (4) are formed three-dimensionally, or only the main link elements (3) are formed three-dimensionally and the connection link elements (4) are formed two-dimensionally.

3. Medical product (1) according to claim 2, **characterized in that** the main link elements (3) are asymmetrical in the z-direction.

4. Medical product (1) according to claim 2 or 3, **characterized in that** the planar grid structure is curvable in the z-direction.

5. Medical product (1) according to claim 4, **characterized in that** the main link elements (3) predefine a maximum curvature of the planar grid structure by their mutual contact.

6. Medical product (1) according to claim 5, **characterized in that** the connection link elements (4) prevent further movement of the main link elements (3), in particular distancing from each other, upon reaching the predefined maximum curvature.

7. Medical product (1) according to claim 6, **characterized in that** the predefined maximum curvature in the positive z-direction is different from the predefined maximum curvature in the negative z-direction.

8. Medical product (1) according to a preceding claim, **characterized in that** the main link elements (3) and the connection link elements (4) have a self-contained shape.

9. Medical product (1) according to claim 6, **characterized in that** the main link elements (3) are each formed as a framework-shaped, triangular frustum of a pyramid and the connection link elements (4) are each formed as an eyelet-shaped polygon, in particular hexagon.

10. Medical product (1) according to claim 6, **characterized in that** the main link elements (3) are formed as framework-like, preferably elongated, quadrangular pyramids and the connection link elements (4) are formed as a dome-shaped grid shell with at least four arched elements (11) at the base and with correspondingly at least four recesses (6), which are each arranged centrally between two adjacent arched elements (11) and a lower edge of the recess (6) is located at the level of the apex of the arched elements (11).

11. Medical product (1) according to claim 6, **characterized in that** the main link elements (3) each have a first base element (12) formed as an eyelet-shaped polygon, in particular a hexagon, which defines a plane, and a second base element (13) formed as an eyelet-shaped polygon, in particular a hexagon, which extends over at least one plane, which are connected to each other at the corners via orthogonal side edges (8), in particular of different lengths, and the connection link elements (4) are each formed by at least three arched elements (11) connected to each other at the base.

12. Medical product (1) according to claim 6, **characterized in that** the main link elements (3) are each formed by a connection of a first and a second central point (9), which are opposite to each other and are connected with at least four side edges (8) which are bent/curved outwards, in particular with at least two adjacent side edges (8), which have a larger bulge (15) closer to the first central point and with at least two further adjacent side edges (8) which have a larger bulge (15) closer to the second central point (9), and the connection link elements are each formed as two framework-like, closed, quadrangular pyramids which are connected to each other at their lower/bottom side to form a closed body.

## Revendications

1. Produit médical (1), en particulier implant sous forme d'un filet à chaînes destiné à être utilisé pour le traitement, en particulier pour le comblement et/ou la fermeture, d'un défaut osseux, avec une pluralité de maillons individuels (2) qui sont connectés entre eux de sorte que des maillons individuels (2) adjacents les uns des autres sont enchaînés les uns dans les autres, dans lequel
les maillons individuels (2) du filet à chaînes sont divisés en maillons principaux (3) et en maillons de connexion (4), dans lequel les maillons principaux (3) et les maillons de connexion (4) forment, à l'état enchaîné entre eux, une structure en treillis plane dans le plan x-y, **caractérisé en ce que** les maillons de connexion (4) présentent une forme géométrique différente de celle des maillons principaux (3).

2. Produit médical (1) selon la revendication 1, **caractérisé en ce que** les maillons principaux (3) et les maillons de connexion (4) sont tridimensionnels, ou seuls les maillons principaux (3) sont tridimensionnels et les maillons de connexion (4) sont bidimensionnels.

3. Produit médical (1) selon la revendication 2, **caractérisé en ce que** les maillons principaux (3) sont conçus de manière asymétrique dans la direction z.

4. Produit médical (1) selon la revendication 2 ou 3, **caractérisé en ce que** la structure en treillis plane peut être courbée dans la direction z.

5. Produit médical (1) selon la revendication 4, **caractérisé en ce que** les maillons principaux (3) prédéfinissent une courbure maximale de la structure en treillis plane par leur contact mutuel.

6. Produit médical (1) selon la revendication 5, **caractérisé en ce que** les maillons de connexion (4) empêchent un déplacement supplémentaire des maillons principaux (3), en particulier un éloignement les uns des autres, lorsque la courbure maximale prédéfinie est atteinte.

7. Produit médical (1) selon la revendication 6, **caractérisé en ce que** la courbure maximale prédéfinie dans la direction z positive est différente de la courbure maximale prédéfinie dans la direction z négative.

8. Produit médical (1) selon une revendication précédente, **caractérisé en ce que** les maillons principaux (3) et les maillons de connexion (4) présentent une forme fermée sur elle-même.

9. Produit médical (1) selon la revendication 6, **caractérisé en ce que** les maillons principaux (3) sont respectivement conçus comme une pyramide tronquée triangulaire de type charpente, et les maillons de connexion (4) sont respectivement conçus comme un polygone, en particulier un hexagone, en forme d'œillet.

10. Produit médical (1) selon la revendication 6, **caractérisé en ce que** les maillons principaux (3) sont conçus comme des pyramides quadrangulaires, de préférence prolongées, de type charpente et les maillons de connexion (4) sont conçus comme une coque en treillis en forme de coupole avec au moins quatre arcs (11) au niveau de la base et avec au moins quatre évidements (6) correspondants qui sont disposés respectivement au milieu entre deux arcs (11) adjacents, et un bord inférieur de l'évidement (6) se trouve à la hauteur du sommet des arcs (11).

11. Produit médical (1) selon la revendication 6, **caractérisé en ce que** les maillons principaux (3) présentent respectivement un premier élément de base (12) conçu comme un polygone, en particulier un hexagone, en forme d'œillet, lequel élément de base définit un plan, et un second élément de base (13) conçu comme un polygone, en particulier un hexagone, en forme d'œillet, lequel élément de base s'étend sur au moins un plan, qui sont connectés l'un à l'autre au niveau des coins par des bords latéraux (8) orthogonaux, en particulier de longueurs différentes, et les maillons de connexion (4) sont conçus respectivement par l'intermédiaire d'au moins trois arcs (11) connectés entre eux au niveau de la base.

12. Produit médical (1) selon la revendication 6, **caractérisé en ce que** les maillons principaux (3) sont conçus respectivement par l'intermédiaire d'une connexion d'un premier et d'un second point central (9) qui sont opposés l'un à l'autre et sont connectés à au moins quatre bords latéraux (8) pliés/courbés vers l'extérieur, en particulier à au moins deux bords latéraux (8) adjacents, bords latéraux qui présentent une plus grande courbure (15) plus près du premier point central et avec au moins deux bords latéraux supplémentaires (8) adjacents qui présentent un plus grand renflement (15) plus près du second point central (9), et les maillons de connexion sont conçus respectivement comme deux pyramides quadrangulaires fermées de type charpente qui sont connectées entre elles au niveau de leur côté inférieur/fond pour former un corps fermé.
